Europäisches Patentamt

(19) European Patent Office

Office européen des brevets

(11) Veröffentlichungsnummer: **0 110 294**
**B1**

(12) **EUROPÄISCHE PATENTSCHRIFT**

(45) Veröffentlichungstag der Patentschrift: **16.05.90**

(51) Int. Cl.⁵: **A 61 K 37/24**

(21) Anmeldenummer: **83111650.4**

(22) Anmeldetag: **22.11.83**

(54) **Mittel zur Behandlung der Osteoporose.**

(30) Priorität: 24.11.82 DE 3243358

(43) Veröffentlichungstag der Anmeldung:
**13.06.84 Patentblatt 84/24**

(45) Bekanntmachung des Hinweises auf die
Patenterteilung:
**16.05.90 Patentblatt 90/20**

(84) Benannte Vertragsstaaten:
**AT BE CH DE FR GB IT LI NL SE**

(56) Entgegenhaltungen:
**JP-A-57 814 48**

**CHEMICAL ABSTRACTS, Band 97, Nr. 25, 1982,
Seite 909, Nr. 216732r, Columbus, Ohio, US; &
JP - A - 82 81 448 (TOYO JOZO CO., LTD.)
21.05.1982**

**CHEMICAL ABSTRACTS, Band 96, 1982, Seite
538, Nr. 33741c, Columbus, Ohio, US; H.F.
DeLUCA: "Vitamin D in health and disease" &
ADV. HUM. CLIN. NUTR. 1982, 107-30**

(73) Patentinhaber: **Hesch, Rolf Dieter, Prof. Dr.
Gartenweg 12
D-3004 Isernhagen 2 (DE)**

(72) Erfinder: **Hesch, Rolf Dieter, Prof. Dr.
Gartenweg 12
D-3004 Isernhagen 2 (DE)**

(74) Vertreter: **Vossius & Partner
Siebertstrasse 4 P.O. Box 86 07 67
D-8000 München 86 (DE)**

(56) Entgegenhaltungen:

CHEMICAL ABSTRACTS, Band 93, Nr. 3, 1980,
Seite 576, Nr. 24876m, Columbus, Ohio, US; J.

GYARMATI Jr. u.a.: "Application of radiological
methods in xperimental endocrine
osteopathies" & STUD. BIOPHYS. 1979, 77(2),
149-50

CHEMICAL ABSTRACTS, Band 93, Nr. 3, 1980,
Seite 576, Nr. 24877n, Columbus, Ohio, US; M.

BULLA u.a.: "Effect of vitamin D3 therapy on
renal osteodystrophy in children" & KLIN.

WOCHENSCHR. 1980, 58(5), 237-47
Römpps Chemie-Lexikon, 7. Auflage (1974)
"Parathormon", S. 2509

Die Akte enthält technische Angaben, die nach
dem Eingang der Anmeldung eingereicht
wurden und die nicht in dieser Patentschrift
enthalten sind.

**Beschreibung**

Die Osteoporose ist die am häufigsten vorkommende Erkrankung des Skeletts und des Halteapparats des Menschen. Die Osteoporose ist keine einheitliche Erkrankung, sondern eine monoforme Reaktion des Skeletts auf eine Vielzahl von möglichen Ursachen. Sie resultiert aus einem Mißverhältnis des Knochenumbaus, bei dem entweder der Knochenanbau vermindert ist bei konstantem Knochenabbau, der Knochenanbau gleichbleibend ist ver vermehrtem Knochenabbau oder der Knochenbau weniger gesteigert ist als der Knochenabbau. Die häufigste Form der Osteoporose äußert sich darin, daß die den Knochenumbau betreffenden Prozesse darniederliegen, die sog. "low-turn-over-Osteoporose". Die Entstehung und Verhinderung dieser Form ist unter den Experten besonders strittig, da ihr wahrscheinlich primäre Störungen zugrunde liegen, die schon über Jahre umbemerkt wirksam gewesen sind.

Grundeinheit des Knochenstoffwechsels ist die BMU (basic multicellular unit) nach Frost—Treatment of osteoporosis by manipulation of coherent bone cell populations, Clin. Orthop. and Relat. Res. 143 (1979), S. 227. Normalerweise erfolgt dort eine geordnete celluläre Tätigkeit von Osteoblasten und Osteoklasten, wahrescheinlich in direkter Informationskopplung zu den tiefer liegenden Osteozyten. Diese Zellen sind in einem gekoppelten Stoffwechsel beider Zellpopulationen zum Knochenanbau und Knochenabbau verbunden. Durch das externe Milieu in der BMU wird deren Stoffwechsel sowie deren Regeneration geregelt. Wesentlicher Regulator des BMU ist das Parathormon; vergl. Parsons, Handbuch der Inneren Medizin, Band 4, Klinische Osteologie, Springer Verlag, Berlin, 1980, S. 135. Dosisabhängig kommt es hierbei zu einer prädominanten Stimulation von Osteoblasten und/oder gekoppelt Osteoklasten. Es ist noch nicht geklärt, ob Parathormon dabei über eine zunächst osteodestruktiv nach nicht wirksame Stimulation der Osteoklasten, die Osteoblasten stimulieren kann oder direkt an den Osteoblasten wirken kann. Die Bedeutung der anderen am Knochenstoffwechsel beteiligten Hormone ist zwar anerkannt, die regulatorische Interaktion dieser Hormone mit den Zellen unter den physiologischen und pathophysiologischen Bedingungen ist aber noch nicht erfasst. Alle bischerigen Versuche, einen darniederliegenden Knochenstoffwechsel, unabhängig von der primären Ursache hierfür, wieder in Gang zu setzen, sind bisher entmutigend verlaufen.

Es ist bereits versucht worden, mit synthetischem humanem (1—34) Parathormon-Fragment den BMU zu stimulieren. Es wurden hierbei täglich subcutane Injektionen von 450—750 Einheiten (1—34) Parathormon-Fragment kontinuierlich appliziert, und zwar über eine Dauer von 6 bis 24 Monaten. Dabei wurden aber nicht nur der Knochenanbau, sondern auch der Knochenabbau in unvorhersehbarer Weise stimuliert, wobei das Ungleichgewicht bei den einzelnen Patienten nicht vorhergesagt werden konnte. Ferner wurde die Bildung von Antikörpern gegen N-terminales Parathormon beobachtet. Außer dem (1—34) Parathormon-Fragment sind auch kürzere sowie modifizierte Parathormonanaloge sowie längere Parathormonpeptide biologisch wirksam. Die Hauptproblematik besteht drain, den für den jeweiligen Zweck geeigneten Bioassay zu finden, da in vivo- und in vitro-Studien Speziesunterschiede und unterschiedliche Target-Antworten nicht berücksichtigen. Die Literatur ist daher voll von verwirrenden und abweichenden Resultaten über die biologische Aktivität unterschiedlicher Parathormonpeptide; vergl. Rosenblatt, Parathyroid hormone, Chemistry and structure-activity relations, Pathobiology Annual, Bd. 11 (1981), S. 53.

Der Erfindung liegt die Aufgabe zugrunde, einen therapeutischen Wirkstoff zur Anwendung bei der Osteoporose bereitzustellen, der in kontrollierbarer und vorhersehbarer Weise den Knochenaufbau stimuliert und den Knochenabbau reguliert.

Diese Aufgabe wird überraschenderweise gelöst durch das humane (1—38) Parathormon-Fragment. Die Erfindung betrifft somit die in den Patentansprüchen 1 bis 4 gekennzeichneten Gegenstände.

Humanes (1—38) Parathormon und ein Verfahren zu seiner Herstellung ist in der japanischen Offenlegungsschrift 81448/1982 beschrieben. In dieser Offenlegungsschrift wird weiterhin die Verwendung dieses Parathormon-Fragments zur Herstellung von Antikörpern beschrieben, die gegen h-PTH gerichtet sind. Als Verwendung der neuen Substanz wird daher ausschließlich angegeben, daß es zur Herstellung von Antikörpern zu diagnostischen Zwecken verwendbar ist. Überraschenderweise wurde jetzt gefunden, daß das humane (1—38) Parathormon-Fragment im Gegensatz zum humanen (1—34) Parathormon-Fragment ausgezeichnet geeignet ist, Osteoporose zu behandeln, weil bei klinischen Langzeituntersuchungen die Bildung von Antikörpern gegen N-terminales Parathormon-Fragment nicht beobachtet werden konnte.

Das humane (1—38) Parathormon-Fragment wird in an sich bekannter Weise hergestellt, indem man in einen Peptidsyntheseautomaten schrittweise auf einem chlormethylierten Harz die Peptidsequenz 1—38 des humanen Parathormons aufbaut, anschließend mit wasserfreier HF in Gegenwart von Anisol vom Trägerharz sowie den mitgeführten Schutzgruppen abspaltet und durch Extraktion und Chromatographie reinigt.

Die Kopplung der Peptidbausteine wird vorzugsweise mit folgenden Reagenzien und Reaktionsschritten durchgeführt:

1. $CH_2Cl_2$ waschen (2 mal)
2. MeOH waschen (2 mal)
3. $CH_2Cl_2$ waschen (3 mal)
4. 50% TFA+5% 1,2 Ethandithiol in $CH_2Cl_2$
5. $CH_2Cl_2$ waschen (2 mal)
6. MeOH waschen (1 mal)

7. Et$_3$N 10% in CH$_2$Cl$_2$+5% DMF
8. MeOH waschen (1 mal)
9. Et$_3$N 10% in CH$_2$Cl$_2$+5% DMF
10. MeOH waschen (1 mal)
11. CH$_2$Cl$_2$ waschen (2 mal)
12. Benzyloxycarbonyl-Aminosäure (20 mmol) in 40 ml CH$_2$Cl$_2$+DCCl (20 mmol) (1 mal)

Zur Ankopplung des jeweils nächsten Peptidbausteins wird diese Reaktionsfolge wiederholt.

Bei dem Verfahren werden die ε-Aminogruppen von Lysin mit der 2-Chlorobenzyloxycarbonylgruppe, von Asparagin und Glutamin mit der Xanthylgruppe geschützt. Die Guanidingruppe von Arginin und die Imidazolgruppe von Histidin wird mit der Tosylgruppe geschützt, und die ß-Carboxylgruppe von Asparaginsäure, die γ-Carboxylgruppe von Glutaminsäure sowie die Hydroxylgruppe von Serin wird mit der Benzylgruppe geschützt.

Die Extraktion erfolgt durch wäßrige Essigsäure, dann Filtrieren über Sephadex$^R$ G-25 mit 1 molarer Essigsäure, Lyophilisieren und Chromatographieren mit Ammoniumacetat-Acetonitril als Elutionsmittel unter Hochdruck.

Das reine Material wird über Sephadex$^R$ G-10 mit 1 molarer Essigsäure entsalzt, das so erhaltene Produkt durch Dünnschicht-Chromatographie in verschiedenen Puffersystemen untersucht und als homogen befunden. Die Aminosäurenanalyse und Sequenzanalyse zeigt das richtige Aminosäureverhältnis. Durch Umkehrphasen HPLC wird gefunden, daß das so erhaltene Produkt zu 98% rein ist. Das so erhaltene Produkt wird steril in Ampullen gefüllt und auf Pyrogenität getestet. Die Stabilität und biologische Aktivität werden in einem Bioassay an humanen Nierenmembranen gemessen gegen den humanen MRC-Standard (London) 79/500 und gegen die bovinen Standard des MRC (London) 67/342, und zwar in gleicher Weise 6000 bis 7000 E/mg bei zwei Auflösungen. Das Material ist am Knochenzell-Assay ebenso wirksam wie das (1—34) Parathormon-Fragment. Bezüglich der Osteoklasten-Stimulation ist das Material äquipotent mit (1—84) extraktivem bovinen Parathormon.

Während, unter und nach der Therapie sollen alle Parameter des parathormonabhängigen Calciumstoffwechsels des Knochens, des Darms und der Niere inklusive der 1.25-(OH)$_2$-Vitamin D-Synthese sorgfältig überwacht werden, um festzustellen, ob die Therapie bei dem jeweiligen Patienten anschlägt und zu keinen Entgleisungen des Stoffwechsels führt.

Das (1—38) Parathormon-Fragment wird vorzugsweise zu einem Injektionspräparat konfektioniert. Die Tagesdosis beträgt 300 bis 600 Einheiten. Zur Unterstützung der Therapie ist es möglich, das (1—38) Parathormon-Fragment zusätzlich und phasenverschoben mit dem den Knochenbau regulierenden EHDP (Ethylenhydroxiddiphosphonat) anzuwenden. Es empfiehlt sich, nach zwei Wochen Therapie mit den synthetischen humanen (1—38) Parathormon-Fragment nach ca. 1 Woche eine dreiwöchige Behandlung mit 2—4, vorzugsweise 2,5 mg/kg EHDP folgen zu lassen. Danach sollten ca. 3 Wochen keine Behandlungen erfolgen und erst dann der nächste Therapie-Schub zur Anwendung kommen.

**Patentansprüche für die Vertragsstaaten: BE, CH, LI, DE, FR, GB, IT, NL, SE**

1. (1—38) Parathormon-Fragment zur Anwendung als therapeutischer Wirkstoff.

2. (1—38) Parathormon-Fragment zur Anwendung bei der Osteoporose.

3. (1—38) Parathormon-Fragment in Kombination mit Ethylenhydroxiddiphosphonat zur Anwendung bei der Osteoporose.

4. Arzneimittel, enthaltend (1—38) Parathormon-Fragment nach einem der Ansprüche 1 bis 3 und ein pharmazeutisch inertes Excipiens.

**Patentansprüche für den Vertragsstaat: AT**

1. Verfahren zur Herstellung eines humanen (1—38) Parathormon-Fragments, dadurch gekennzeichnet, daß die Peptidsequenz 1—38 des humanen Parathormons schrittweise auf einem chlormethylierten Harz aufgebaut, anschließend mit wasserfreier HF in Gegenwart von Anisol vom Trägerharz sowie den mitgeführten Schutzgruppen abgespalten und Extraktion und Chromatographie gereinigt wird.

2. Verfahren nach Anspruch 1, dadurch gekennzeichnet, daß die Kopplung der einzelnen Peptidbausteine je Baustein mit folgenden Reagenzien und gemäß den folgenden Reaktionsschritten durchgeführt wird:

1. CH$_2$Cl$_2$ waschen (2 mal)
2. MeOH waschen (2 mal)
3. CH$_2$Cl$_2$ waschen (3 mal)
4. 50% TFA+5% 1,2 Ethandithiol in CH$_2$Cl$_2$
5. CH$_2$Cl$_2$ waschen (2 mal)
6. MeOH waschen (1 mal)
7. Et$_3$N 10% in CH$_2$Cl$_2$+5% DMF
8. MeOH waschen (1 mal)
9. Et$_3$N 10% in CH$_2$Cl$_2$+5% DMF
10. MeOH waschen (1 mal)
11. CH$_2$Cl$_2$ waschen (2 mal)
12. Benzyloxycarbonyl-Aminosäure (20 mmol) in 40 ml CH$_2$Cl$_2$+DCCl (20 mmol) (1 mal)

3. Verfahren nach Anspruch 1 oder 2, dadurch gekennzeichnet, daß die ε-Aminogruppen von Lysin mit einer 2-Chlorobenzyloxycarbonylgruppe und von Asparagin und Glutamin mit einer Xanthylgruppe geschützt werden.

4. Verfahren nach einem der Ansprüche 1 bis 3, dadurch gekennzeichnet, daß die Guamidingruppe von Arginin und die Imidazolgruppe von Histidin mit einer Tosylgruppe geschützt werden.

5. Verfahren nach einem der Ansprüche 1 bis 4, dadurch gekennzeichnet, daß die ß-Carboxylgruppe von Asparaginsäure und die γ-Carboxylgruppe von Glutaminsäure sowie die Hydroxylgruppe von Serin mit einer Benzylgruppe geschützt werden.

6. Verfahren nach einem der Ansprüche 1 bis 5, dadurch gekennzeichnet, daß zur Reinigung die Extraktion mit wässeriger Essigsäure vorgenommen wird, wonach dann die wirkstoffhältige Phase über Sephadex G-25 mit 1 molarer Essigsäure filtriert, lyophilisiert und danach mit Ammoniumacetat und Acetonitril als Elutionsmittel unter Hochdruck chromatographiert wird.

7. Verfahren zur Herstellung eines (1—38) Parathormon-Fragments enthaltenden Arzneimittels, dadurch gekennzeichnet, daß das nach einem der Ansprüche 1 bis 6 hergestellte (1—38) Parathormon-Fragment mit herkömmlichen Excipienten zu einer Injektionslösung konfektioniert wird.

**Revendications pour les Etats Contractants: BE, CH, LI, DE, FR, GB, IT, NL, SE**

1. Fragment (1—38) de la parathormone destiné à être utilisé comme substance active thérapeutique.

2. Fragment (1—38) de la parathormone destiné à être utilisé dans le cas de l'ostéoporose.

3. Fragment (1—38) de la parathormone en combinaison avec le diphosphonate de l'hydroxyde d'éthylène destiné à être utilisé dans le cas de l'ostéoporose.

4. Médicament contenant le fragment (1—38) de la parathormone selon l'une des revendications 1 à 3 et un excipient inerte du point de vue pharmaceutique.

**Revendications pour l'Etat Contractant: AT**

1. Procédé de préparation d'un fragment (1—38) de la parathormone humaine, caractérisé en ce que la séquence peptidique 1—38 de la parathormone humaine est constituée par étapes sur une résine chlorométhylée, puis est séparée de la résine de support et des groupes protecteurs entraînés avec HF anhydre en présence d'anisol et est purifiée par extraction et chromatographie.

2. Procédé selon la revendication 1, caractérisé en ce que le couplage des différents éléments constitutifs peptidiques est réalisé pour chaque élément constitutif avec les réactifs suivants et selon les étapes réactionnelles suivantes:

1. $CH_2Cl_2$ laver (2 fois)
2. MeOH laver (2 fois)
3. $CH_2Cl_2$ laver (3 fois)
4. 50% TFA+5% 1,2 éthanedithiol dans $CH_2Cl_2$
5. $CH_2Cl_2$ laver (2 fois)
6. MeOH laver (1 fois)
7. $Et_3N$ 10% dans $CH_2Cl_2$+5% DMF
8. MeOH laver (1 fois)
9. $Et_3N$ 10% dans $CH_2Cl_2$+5% DMF
10. MeOH laver (1 fois)
11. $CH_2Cl_2$ laver (2 fois)
12. Acide benzyloxycarbonylaminé (20 mmol) dans 40 ml $CH_2Cl_2$+DCCI (20 mmol) (1 fois)

3. Procédé selon la revendication 1 ou 2, caractérisé en ce que les groupes amine ε de la lysine sont protégés avec un groupe 2-chlorobenzyloxycarbonyle et ceux de l'asparagine et de la glutamine avec un groupe xanthyle.

4. Procédé selon l'une des revendications 1 à 3, caractérisé en ce que le groupe guamidine de l'arginine et le groupe imidazole de l'histidine sont protégés avec un groupe tosyle.

5. Procédé selon l'une des revendications 1 à 4, caractérisé en ce que le groupe carboxyle β de l'acide aspartique et le groupe carboxyle γ de l'acide glutamique ainsi que le groupe hydroxyle de la sérine sont protégés avec un groupe benzyle.

6. Procédé selon l'une des revendications 1 à 5, caractérisé en ce que l'extraction avec de l'acide acétique aqueux est entreprise en vue de la purification, après quoi la phase contenant la substance active est filtrée sur du Sephadex G—25 avec de l'acide acétique une fois molaire, lyophilisée et ensuite chromatographiée sous haute pression avec l'acétate d'ammonium et l'acétonitrile comme éluants.

7. Procédé de préparation d'un médicament contenant le fragment (1—38) de la parathormone, caractérisé en ce que le fragment (1—38) de la parathormone préparé selon l'une des revendications 1 à 6 est mis sous forme d'une solution d'injection avec des excipients habituels.

**Claims for the Contracting States: BE, CH, LI, DE, FR, GB, IT, NL, SE**

1. A (1—38) parathyroid hormone fragment for the application as a therapeutically active ingredient.

2. A (1—38) parathyroid hormone fragment for the application in the treatment of osteoporosis.

3. A (1—38) parathyroid hormone fragment in combination with ethylene hydroxide diphosphonate for the application in the treatment of osteoporosis.

4. A pharmaceutical preparation which contains a (1—38) parathyroid hormone fragment according to any one of the Claims 1 to 3 and a pharmaceutically inert excipient.

**Claims for the Contracting State: AT**

1. A process for the production of a human (1—38) parathyroid hormone fragment, characterized by synthesizing the peptide sequence 1—38 of said human parathyroid hormone in a stepwise manner on a chloromethylated resin, subsequently cleaving it from the carrier resin and from the concomitant protective groups by means of anhydrous HF in the presence of anisole, and purifying it by extraction and chromatography.

2. The process of Claim 1, characterized by carrying out the coupling of the individual peptide units per unit with the following reagents and according to the following reaction steps:

1. $CH_2Cl_2$ wash (2 times)
2. MeOH wash (2 times)
3. $CH_2Cl_2$ wash (3 times)

4. 50% TFA+5% 1,2-ethanedithiol in $CH_2Cl_2$
5. $CH_2Cl_2$ wash (2 times)
6. MeOH wash (once)
7. $Et_3N$ 10% in $CH_2Cl_2$+5% DMF
8. MeOH wash (once)
9. $Et_3N$ 10% in $CH_2Cl_2$+5% DMF
10. MeOH wash (once)
11. $CH_2Cl_2$ wash (2 times)
12. Benzyloxycarbonylamino acid (20 mmoles) in 40 ml $CH_2Cl_2$+DCCl (20 mmoles) (once).

3. The process of Claim 1 or 2, characterized by protecting the ε-amino groups of lysine with a 2-chlorobenzyloxycarbonyl group and those of asparagine and glutamine with a xanthyl group.

4. The process of any one of the Claims 1 to 3, characterized by protecting the guanidine group of arginine and the imidazole group of histidine with a tosyl group.

5. The process of any one of the Claims 1 to 4, characterized by protecting the β-carboxy group of aspartic acid and the γ-carboxy group of glutamic acid as well as the hydroxy group of serine with a benzyl group.

6. The process of any one of the Claims 1 to 5, characterized by carrying out the extraction for purifying with aqueous acetic acid, subsequently filtering the phase containing the active ingredient over Sephadex G-25 with 1-molar acetic acid, lyophilizing it and subsequently chromatographing it under high pressure using ammonium acetate and acetonitrile as eluting agents.

7. A process for producing a pharmaceutical preparation containing a (1—38) parathyroid hormone fragment which comprises producing an injection solution from the (1—38) parathyroid hormone fragment prepared according to any one of the Claims 1 to 6 and conventional excipients.